# EUROPEAN PATENT SPECIFICATION

(11) **EP 2 875 835 B1**
(45) Date of publication and mention of the grant of the patent: **28.12.2016**
(21) Application number: 14193999.1
(22) Date of filing: 20.11.2014
(51) Int. Cl.: A61M 1/06

(54) **MANUAL BREAST PUMP**
HANDBETÄTIGTE BRUSTPUMPE
TIRE-LAIT MANUEL

(30) Priority: 25.11.2013 IT MI20131960
(43) Date of publication of application: 27.05.2015
(73) Proprietor: Artsana S.p.A., 22070 Grandate (CO) (IT)
(72) Inventor: Bianchi, Andrea, 22070 Grandate CO (IT); Gatti, Francesco, 22070 Grandate CO (IT)
(74) Representative: Perani, Aurelio

(56) References cited:
- EP-A1- 2 708 248
- EP-A2- 0 733 376
- US-A- 50 457
- US-A1- 2003 204 164
- US-A1- 2007 191 763
- US-A1- 2010 262 072

## Description

The present invention relates to a breast pump as defined in the preamble of the main claim.

Breast pumps designed to be operated by a single hand of a user have been long known. For example, EP733376 discloses a single-hand operable breast pump comprising a body with a portion adapted to be coupled with a portion of the breast of a user. A conduit in the breast pump body is connected to such portion and opens into an end portion of such body, which is adapted to be removably connected to a container; the latter receives breast expressed milk.

A valve is placed in such conduit, for generating an appropriate pressure in the conduit and hence in the funnel-shaped portion, for milk to be expressed and conveyed into the container Such expression is obtained by means of a diaphragm member, which is sealably and movably held in a specially designed chamber of the breast pump body, under the action of a lever hinged to such body. The chamber communicates with the above mentioned conduit and the movement of the diaphragm member causes the generation of negative pressure waves, allowing milk to be expressed and conveyed to the container.

While prior art arrangements allow single-hand operation of the breast pump, for the other hand to hold the milk container, they still have a non-negligible problem associated with the start of the movement of the diaphragm member in its chamber, when breast milk expression is started.

This is because the diaphragm member usually operates as a piston which can move parallel to itself (i.e. along a center axis orthogonal thereto) within the chamber of the breast pump body, which has a cylinder-like shape. This member or piston comprises a joining element which orthogonally raises therefrom at a center portion and has the actuation lever connected thereto, which is in turn hinged to the breast pump body such that its movement causes the above mentioned movement of the diaphragm member, parallel to itself, in its chamber. When the diaphragm member is at rest, it lies on a bottom portion of the above mentioned chamber in communication with the the conduit connected to the funnel-shaped element and a pressure (atmospheric pressure) is distributed over its entire surface (opposite to the side with which it lies on such bottom portion).

When suction is started, the woman or user acting on the lever must overcome the pressure that progressively builds up in the conduit, "sealed" by the breast, and that tends to "stick" the diaphragm member on the bottom of the above mentioned chamber, which causes the user to force its action upon the lever to start such suction. As a result, the woman will naturally press the breast pump toward its breast and may feel pain. In addition, the initial effort on the lever to cause it to separate from the bottom of the chamber may cause a loss of grip on the lever, especially if the milk container already contains liquid; this may cause the latter to fall, which will involve apparent drawbacks.

Examples of breast pumps are known from US2003/204164, US2010/262072, US2007/191763, EP0733376 and EP2708248.

A further breast pump is disclosed in US 50457 which represents the closest prior art with respect to appended claim 1.

The object of the present invention is to provide an improved breast pump as compared with the prior art.

Particularly, the object of the invention is to provide a breast pump whose operation is smoother and simpler for the user.

Another object is to provide a breast pump that requires a smaller lever actuation force as compared with prior art arrangements having the same suction performances.

A further object is to provide a breast pump that has a small number of parts and a low cost.

These and other objects, that will be apparent to the skilled person, are fulfilled by a breast pump as defined in the annexed claims.

The present invention will be better understood with reference to the accompanying drawings, which are given by way of example and without limitation, in which:
Figure 1 shows a perspective view of a breast pump of the invention;
Figure 2 shows an exploded perspective view of the breast pump of Figure 1;
Figure 3 shows a cross sectional view taken along line 3-3 of Figure 1;
Figure 4 shows a cross sectional view like that of Figure 3, but with the breast pump in a different operating position;
Figure 5 shows a perspective view of a variant of the breast pump of Figure 1;
Figure 6 shows a cross sectional view taken along line 6-6 of Figure 5; and
Figure 7 shows a cross sectional view like that of Figure 7, but with the breast pump in a different operating step.

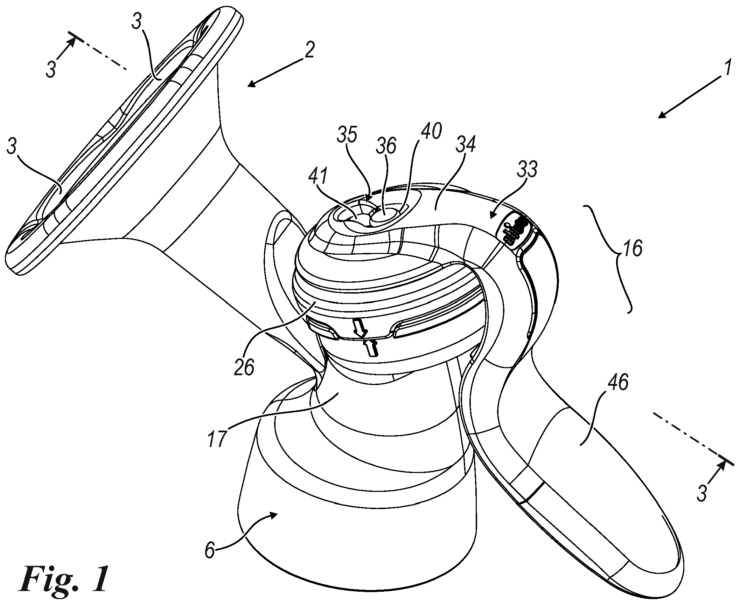

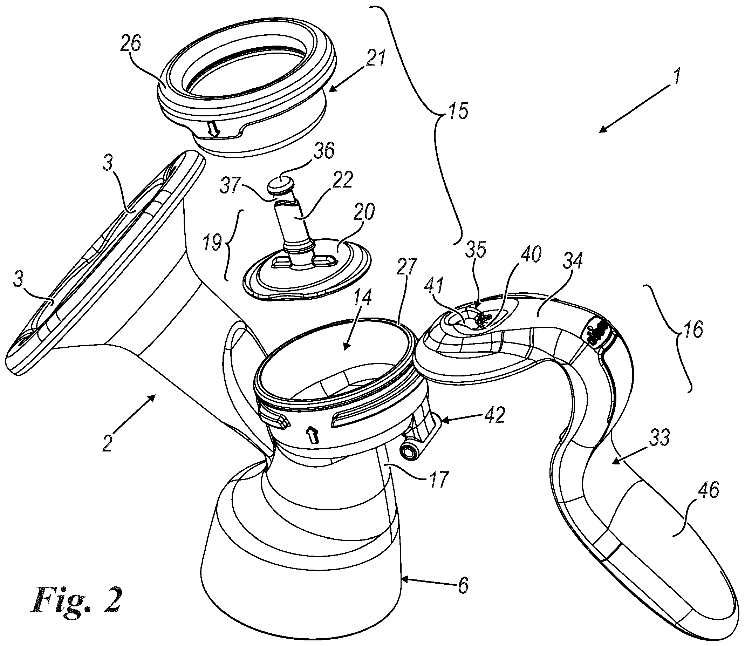

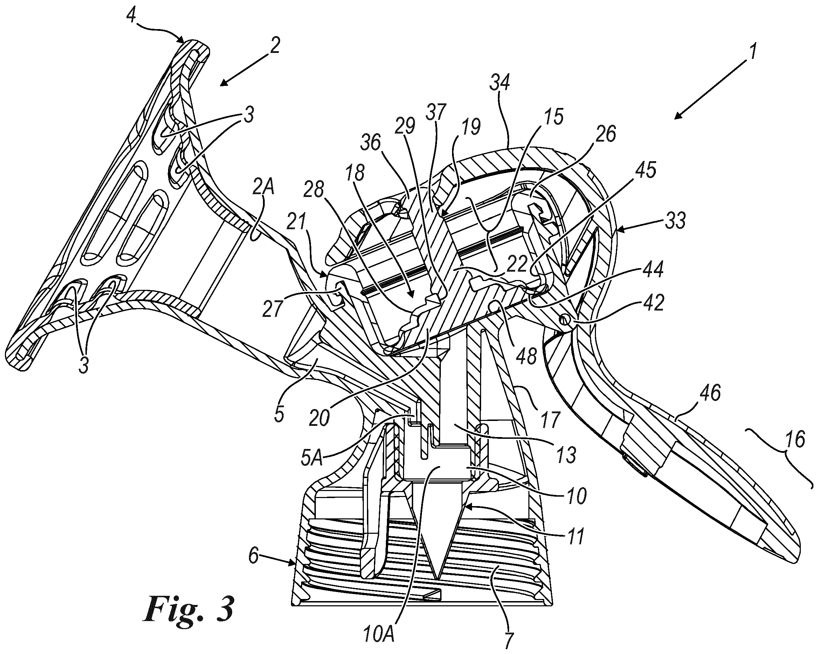

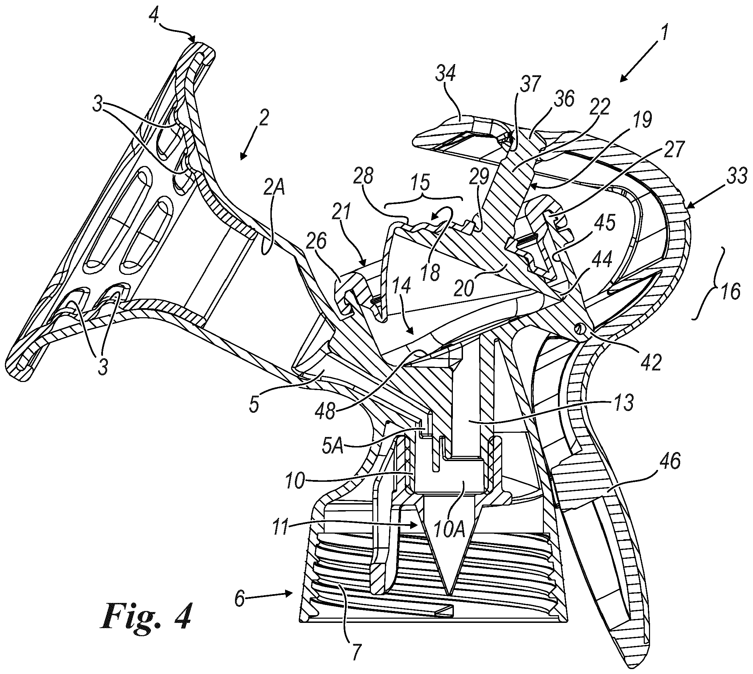

Referring to the above mentioned figures, and particularly to Figures 1-4, a first embodiment of a breast pump of the invention is shown. It is adapted to be operated by a single hand of a user, and comprises a body 1 having a funnel-shaped portion 2 for receiving at least part of the breast of a user. Preferably, such funnel-shaped portion 2 may be coupled to a sleeve 4 made of a soft material, which is fitted into an inner wall 2A by the user herself, as shown in Figures 3 and 4, and comprises usual ridges 3 for ensuring comfortable support of the breast.

The funnel-shaped portion 2 is connected to a conduit 5, which is formed in the body 1 to terminate at an end portion 6 of such body, to allow the latter to be joined to a container (not shown) for collection of the expressed milk. For this purpose, this portion 6 is configured as a socket with internal threads 7, allowing it to be tightened on the container.

Particularly, one end 5A of the conduit 5 terminates into a socket portion 10 of the body 1 (close to the end portion 6) on which a lip or duckbill valve 11 is fitted and protrudes into said portion 6, said valve substantially closing the above mentioned end 5A.

A suction channel 13 is connected to a cavity 10A of said socket portion (connected to the conduit 5) and opens into a suction chamber 14 which contains suction means 15 actuated by actuator means 16, the latter being hinged to an outer wall 17 if the body 1. These suction means 15 comprise a diaphragm member 18 moving in said chamber.

More particularly, such member 18 is in turn coupled to a rigid body 19 having a disk-shaped portion 20 coupled to a flexible body 21 (which covers the portion 20) and comprising a shaft 22 orthogonally extending from such portion and acting as a connection between the diaphragm member 18 and the actuator means 16.

The flexible body 21 is configured as a socket and comprises a free edge 26 which is turned up or fitted onto a peripheral edge 27 of the suction chamber 14 and is this fixed to the body 1 of the breast pump. The body 21 comprises an end portion 28, having a hole 29, which is fitted onto the portion 20 of the rigid body 18, the shaft 22 extending through the hole 29.

Such shaft 22 has actuator means 16 associated therewith, which are defined by a lever 33 having an end head 34 with an elongated hole 35 formed therein, and designed to be coupled with such shaft. Particularly, the latter has an end of end head 36 supported by a neck 37 having a smaller cross section than the rest of the shaft. This neck can be placed next to a narrower portion 40 of the elongated hole 35, a wider portion 41 thereof receiving the end 36 of the shaft when the breast pump is assembled. During such operation, the end head 34 of the lever 33 is placed with the wider portion 41 of the hole 35 above the shaft 22, for the end 36 of the latter to be introduced therein; then the neck 37 is moved into the narrower portion 40 of the hole 35 while coupling the lever to the shaft 22, as the end 36 of the latter is wider than the above mentioned portion 40 and cannot fit therethrough.

The lever 33 is hinged to the breast pump body 1 at a hinge 42 incorporated in and external to one side of the chamber 14, below the latter, i.e. in a position located between the chamber 14 and the end portion 6 of the body 1.

The diaphragm member 18 is designed to rotate at a contact area 44 on an edge of its disk-shaped portion 20 that abuts a wall (inner wall) 45 of the chamber 14, when the lever 33 is pushed by acting upon a handle thereof 46 toward the body 1. Such rotation against the wall 45 causes the disk-shaped portion to raise from a bottom or bottom wall 48 of the chamber 14 having the suction channel 13 opening therein, and such channel transfers the suction action of the member 18 to the conduit 5 and then to the breast through the funnel-shaped portion 2 (and the valve 11 closes at the same time). As the portion 20 moves back to the above mentioned bottom 48, it generates a pressure in the channel 13, which pushes the breast-expressed milk into the container coupled to the end portion 6 of the body 1, as the valve 11 opens. The movement of the portion 20 toward the bottom is facilitated by the inherent elasticity of the flexible body 21, which is made of rubber, silicone or the like.

Since the diaphragm member 18 rotates in the chamber 14 while abutting the wall thereof, according to the length of the shaft the force required for such rotation to be applied to the lever 33 is lower than that applied to the latter when it operates a diaphragm member that moves parallel to itself along an axis coinciding with the longitudinal axis of the shaft 22. This is because, in the latter case, the lifting force must overcome a pressure that acts over the entire surface of the portion 20 of the member 18, whereas in the present invention, a moment has to be generated which is proportional to the force arm that tends to rotate such member, said arm being equal to the distance between the point of rotation of said member on the wall 45 of the chamber 14 and the point

(hole 35) at which the shaft 22 is joined to the head 34 of the lever where the rotation force is generated. The longer the shaft 22, the lower the force to be applied to cause the member 18 to rotate on the wall 45.

Due to the "hinge" created between the member 18 and the wall 45 (i.e. the contact between the area 44 and the wall 45) and to the abutment of such member on said wall, the force applied by the user to the lever 33 for milk expression is lower than in prior art arrangements, which will enhance user-friendliness of the breast pump and facilitate breast milk expression.

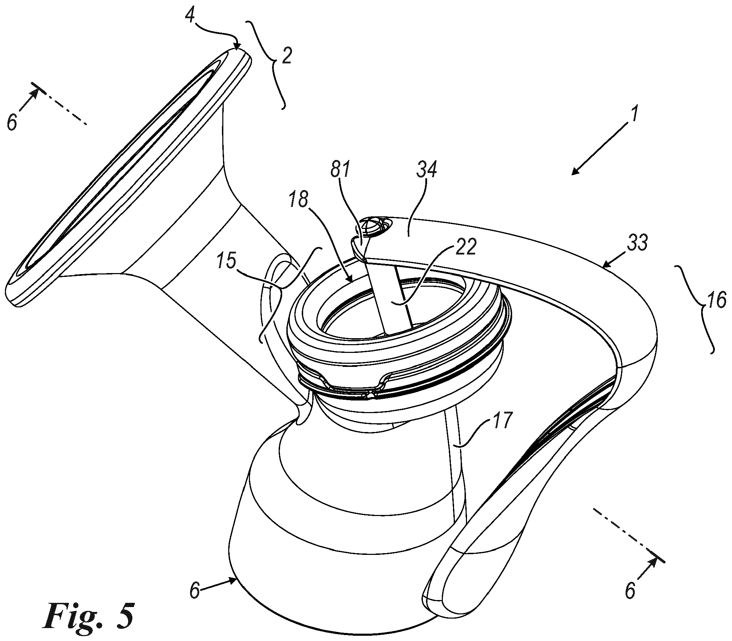

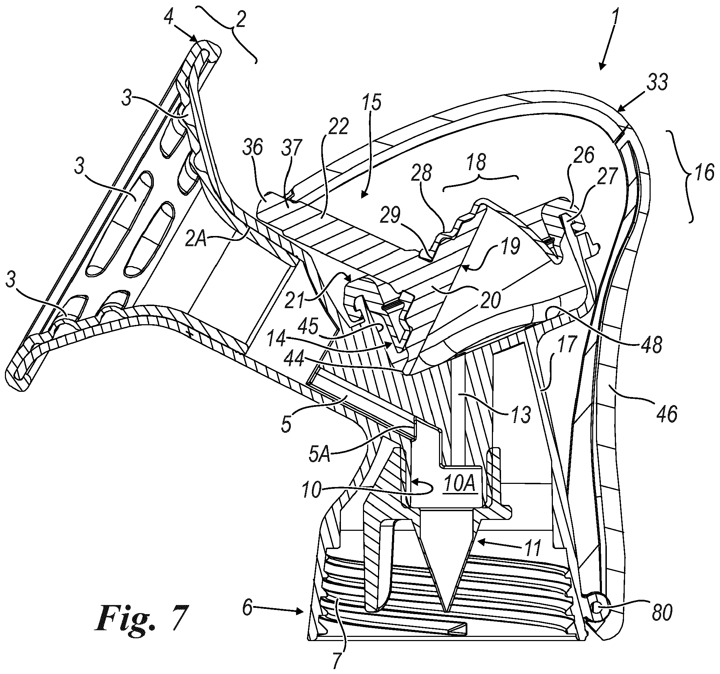

Figures 5 to 7 show a variant of the invention. These figures, in which the parts that have been already described with reference to Figures 1 to 4 are designated by the same numerals, show a breast pump in which the lever 33 is hinged at 80 to the body 1 at the end portion 6. In this arrangement, the diaphragm member 18 is operated by rotating it toward the funnel-shaped portion 2 within the chamber 14 (unlike the arrangement of Figures 1 to 4, in which such member rotates away from such portion 2).

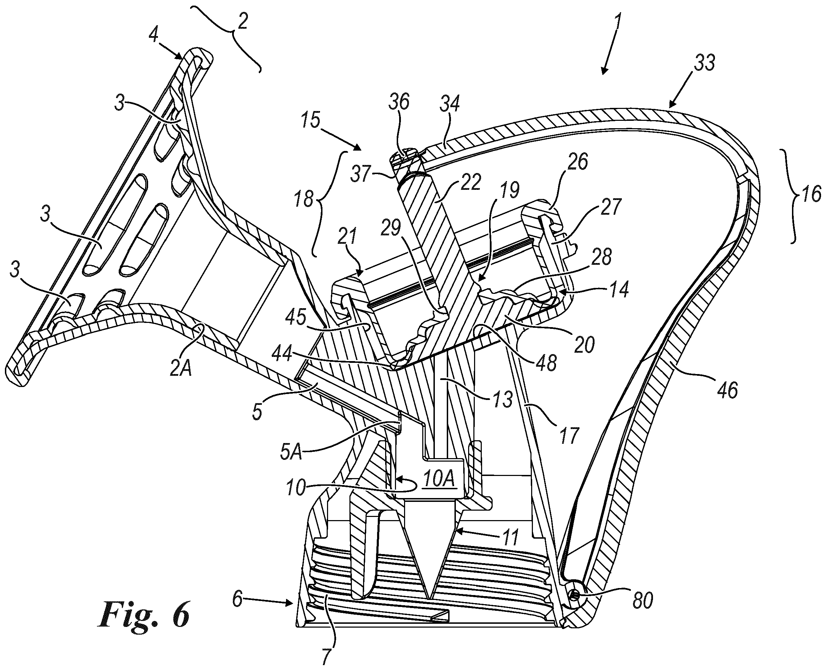

In addition, in the arrangement of Figures 6 to 8, the head 34 of the lever 33 has the hole 35 open on one side adapted for snap engagement (by elastic deformation of the fork portion 81 of the head 34 which bounds such hole) with the neck 37 of the shaft 22.

In this arrangement also, the movement of the member 18 is facilitated (as compared with prior art arrangements) as it rotates on the wall 45 of the chamber 14.

Different embodiments of the invention have been described hereinabove. More embodiments may be also envisaged when considering the above description, and shall be intended to fall within the scope defined by the claims hereinbelow.

## Claims

1. A breast pump adapted to be operated by a single hand of a user, comprising a body (1) having a funnel-shaped portion (2) adapted to receive at least part of the breast of a user and connected to a conduit (5), which is adapted to transfer the breast-expressed milk to an end portion (6) of such body (1), the latter being connected to a container, to collect such liquid therein, said body (1) comprising suction means (15) actuated by actuator means (16), which are adapted to allow milk to be expressed and introduced into said container by an action of the user on said actuator means, said suction means (15) comprising a diaphragm member (18) moving in a suction chamber (14) which is functionally connected to said conduit (5) and contained in said breast pump body (1), said diaphragm member being adapted to rotate about a contact area (44) thereof, which abuts a wall (45) of such suction chamber (14) as a result of the action of the user upon the actuator means (16), **characterized in that** the actuator means (16) comprise an actuating lever (33) which is hinged to an outer wall (17) of the breast pump body (1) in a position below the suction chamber (14).

2. A breast pump as claimed in claim 1, **characterized in that** the actuating lever (33) is hinged (at 42) external to and on one side of the suction chamber (14) and below it, the diaphragm member controlled by such lever moving within the suction chamber (14) away from the funnel-shaped portion (2).

3. A breast pump as claimed in claim 1, **characterized in that** the lever (33) is hinged (at 80) at the end portion (6) of the breast pump body (1), the diaphragm member (18) controlled by such lever (33) moving within the suction chamber (14) toward the funnel-shaped portion (2).

4. A breast pump as claimed in claim 1, **characterized in that** the diaphragm member (18) comprises a rigid body (19) coupled to a flexible body (21) which covers a disk-shaped portion (20) of such rigid body, a shaft (22) orthogonally extending therefrom and being coupled to said actuator means (16), said shaft (22) extending through a hole (29) of an end portion (28) of said flexible body (21) adapted to cover said disk-shaped potion (20), said flexible body having a socket shape and comprising a free edge (26) fitted onto a peripheral edge (27) of the suction chamber (14).

5. A breast pump as claimed in claims 1 and 4, **characterized in that** said contact area (44) is an edge area of the disk-shaped portion (20) of the rigid body (19) of the diaphragm member (18).

6. A breast pump as claimed in claim 4, **characterized in that** said shaft (22) has an end head (36) supported by a neck (37) having a narrower section than the rest of the shaft that extends upwards from the disk-shaped portion.

7. A breast pump as claimed in claim 6, **characterized in that** the actuating lever comprises a head (34) having a hole (35) adapted to receive the neck (37) of the shaft (22).

8. A breast pump as claimed in claim 7, **characterized in that** said hole (35) is an elongate hole having a narrower portion (40) adapted to receive the neck of the shaft (22) for moving the diaphragm member (18) and a wider portion (41) for receiving the end head (36) of such shaft (22) for coupling thereto.

9. A breast pump as claimed in claim 7, **characterized in that** said hole (35) is open on one side and bounded by a fork portion (81) of the head (34) of the actuating lever (33).

10. A breast pump as claimed in claim 1, **characterized in that** the suction chamber (14) is connected to the conduit (5) connected to the funnel-shaped portion (2) through a suction channel (13) which is designed to transfer the pressure change generated in said chamber by the movement of the diaphragm member (18) to said conduit to allow milk to be expressed from the breast or conveyed to the container.

11. A breast pump as claimed in claim 10, **characterized in that** said suction channel (13) opens into the bottom wall (48) of the suction chamber.

12. A breast pump as claimed in claim 10, **characterized in that** said conduit (5) and said suction channel (13) open into a cavity (10A) of a portion (10) of the body (1) with which a lip valve is associated and from which the expressed milk flows into the container (6) coupled to the breast pump.

## Patentansprüche

1. Brustpumpe, die dafür angepasst ist, mit einer Hand von einer Benutzerin bedient zu werden, umfassend einen Körper (1) mit einem trichterförmigen Abschnitt (2), der dafür angepasst ist, wenigstens einen Teil der Brust einer Benutzerin aufzunehmen und mit einer Leitung (5) verbunden ist, die dafür angepasst ist, die von der Brust abgepumpte Milch zu einem Endabschnitt (6) eines solchen Körpers (1) zu leiten, wobei letzterer mit einem Behälter verbunden ist, um eine solche Flüssigkeit darin zu sammeln, wobei der Körper (1) eine Saugeinrichtung (15) umfasst, die durch ein Betätigungsmittel (16) betätigt wird, das dafür angepasst ist, zu erlauben, dass die Milch durch Einwirken der Benutzerin auf das Betätigungsmittel abgepumpt und in den Behälter eingeleitet wird, wobei die Saugeinrichtung (15) ein Membranelement (18) umfasst, das sich in der Ansaugkammer (14) bewegt, die mit der Leitung (5) funktionsverbunden und in dem Brustpumpenkörper (1) enthalten ist, wobei das Membranelement dafür angepasst ist, sich um eine Kontaktfläche (44) desselben zu drehen, die an eine Wand (45) einer solchen Ansaugkammer (14) infolge des Einwirkens der Benutzerin auf das Betätigungsmittel (16) anstößt, **dadurch gekennzeichnet, dass** das Betätigungsmittel (16) einen Betätigungshebel (33) umfasst, der an einer äußeren Wand (17) des Brustpumpenkörpers (1) in einer Stellung unterhalb der Ansaugkammer (14) angelenkt ist.

2. Brustpumpe nach Anspruch 1, **dadurch gekennzeichnet, dass** der Betätigungshebel (33) (in 42) außerhalb und auf einer Seite der Ansaugkammer (14) und unterhalb derselben angelenkt ist, wobei das Membranelement, das mit einem solchen Hebel gesteuert wird, sich innerhalb der Ansaugkammer (14) von dem trichterförmigen Abschnitt (2) weg bewegt.

3. Brustpumpe nach Anspruch 1, **dadurch gekennzeichnet, dass** der Hebel (33) (in 80) am Endabschnitt (6) des Brustpumpenkörpers (1) angelenkt ist, wobei das Membranelement (18), das mit einem solchen Hebel (33) gesteuert wird, sich innerhalb der Ansaugkammer (14) zu dem trichterförmigen Abschnitt (2) hin bewegt.

4. Brustpumpe nach Anspruch 1, **dadurch gekennzeichnet, dass** das Membranelement (18) einen starren Körper (19) umfasst, der an einen flexiblen Körper (21) angekoppelt ist, der einen scheibenförmigen Abschnitt (20) eines solchen starren Körpers bedeckt, wobei eine Welle (22) sich orthogonal hiervon erstreckt und an das Betätigungsmittel (16) angekoppelt ist, wobei die Welle (22) sich durch ein Loch (29) eines Endabschnitts (28) des flexiblen Körpers (21) erstreckt, der dafür angepasst ist, den scheibenförmigen Abschnitt (20) zu bedecken, wobei der flexible Körper eine Buchsenform aufweist und einen freien Rand (26) umfasst, der auf einen umlaufenden Rand (27) der Ansaugkammer (14) aufgesteckt ist.

5. Brustpumpe nach den Ansprüchen 1 und 4, **dadurch gekennzeichnet, dass** die Kontaktfläche (44) eine Randfläche des scheibenförmigen Abschnitts (20) des starren Körpers (19) des Membranelements (18) ist.

6. Brustpumpe nach Anspruch 4, **dadurch gekennzeichnet, dass** die Welle (22) einen Endkopf (36) aufweist, der von einem Hals (37) mit einem schmaleren Querschnitt als die übrige Welle gestützt wird, die sich von dem scheibenförmigen Abschnitt nach oben erstreckt.

7. Brustpumpe nach Anspruch 6, **dadurch gekennzeichnet, dass** der Betätigungshebel einen Kopf (34) mit einem Loch (35) umfasst, das dafür angepasst ist, den Hals (37) der Welle (22) aufzunehmen.

8. Brustpumpe nach Anspruch 7, **dadurch gekennzeichnet, dass** das Loch (35) ein Langloch mit einem schmaleren Abschnitt (40) ist, das dafür angepasst ist, den Hals der Welle (22) zum Bewegen des Membranelements (18) aufzunehmen, und einen breiteren Abschnitt (41) zum Aufnehmen des Endkopfes (36) einer solchen Welle (22) zum Ankoppeln hieran.

9. Brustpumpe nach Anspruch 7, **dadurch gekennzeichnet, dass** das Loch (35) auf einer Seite offen und durch einen Gabelabschnitt (81) des Kopfes (34) des Betätigungshebels (33) begrenzt ist.

10. Brustpumpe nach Anspruch 1, **dadurch gekennzeichnet, dass** die Ansaugkammer (14) mit der Leitung (5) verbunden ist, die mit dem trichterförmigen Abschnitt (2) durch einen Ansaugkanal (13) verbunden ist, der ausgestaltet ist, um den in der Kammer durch die Bewegung des Membranelements (18) erzeugten Druckwechsel auf die Leitung zu übertragen, um es der Milch zu erlauben, von der Brust abgepumpt oder in den Behälter geleitet zu werden.

11. Brustpumpe nach Anspruch 10, **dadurch gekennzeichnet, dass** der Ansaugkanal (13) in die Bodenwand (48) der Ansaugkammer einmündet.

12. Brustpumpe nach Anspruch 10, **dadurch gekennzeichnet, dass** die Leitung (5) und der Ansaugkanal (13) in einen Hohlraum (10A) eines Abschnitts (10) des Körpers (1) einmünden, mit dem ein Lippenventil verbunden ist und aus dem die abgepumpte Milch in den an die Brustpumpe angekoppelten Behälter (6) fließt.

## Revendications

1. Tire-lait adapté pour être actionné par une seule main d'une utilisatrice, comprenant un corps (1) ayant une portion en forme d'entonnoir (2) adaptée pour recevoir au moins une partie du sein d'une utilisatrice et connectée à un conduit (5), qui est adapté pour transférer le lait maternel exprimé vers une portion d'extrémité (6) de ce corps (1), ce dernier étant connecté à un récipient, pour recueillir ce liquide à l'intérieur, ledit corps (1) comprenant des moyens d'aspiration (15) actionnés par des moyens d'actionnement (16), qui sont adaptés pour permettre au lait d'être exprimé et introduit dans ledit récipient par une action de l'utilisatrice sur lesdits moyens d'actionnement, lesdits moyens d'aspiration (15) comprenant un organe formant diaphragme (18) mobile dans une chambre d'aspiration (14) qui est connectée fonctionnellement audit conduit (5) et contenue dans ledit corps de tire-lait (1), ledit organe formant diaphragme étant adapté pour tourner autour d'une zone de contact (44) de celui-ci, qui jouxte une paroi (45) de cette chambre d'aspiration (14) en conséquence de l'action de l'utilisatrice sur les moyens d'actionnement (16), **caractérisé en ce que** les moyens d'actionnement (16) comprennent un levier d'actionnement (33) qui est articulé sur une paroi extérieure (17) du corps de tire-lait (1) dans une position au-dessous de la chambre d'aspiration (14).

2. Tire-lait selon la revendication 1, **caractérisé en ce que** le levier d'actionnement (33) est articulé (en 42) à l'extérieur et sur un côté de la chambre d'aspiration (14) et au-dessous de celle-ci, l'organe formant diaphragme commandé par ce levier se déplaçant à l'intérieur de la chambre d'aspiration (14) en s'éloignant de la portion en forme d'entonnoir (2).

3. Tire-lait selon la revendication 1, **caractérisé en ce que** le lever (33) est articulé (en 80) au niveau de la portion d'extrémité (6) du corps de tire-lait (1), l'organe formant diaphragme (18) commandé par ce levier (33) se déplaçant à l'intérieur de la chambre d'aspiration (14) vers la portion en forme d'entonnoir (2).

4. Tire-lait selon la revendication 1, **caractérisé en ce que** l'organe formant diaphragme (18) comprend un corps rigide (19) couplé à un corps flexible (21) qui couvre une portion en forme de disque (20) de ce corps rigide, un arbre (22) s'étendant orthogonalement à partir de celui-ci et étant couplé auxdits moyens d'actionnement (16), ledit arbre (22) s'étendant à travers un trou (29) d'une portion d'extrémité (28) dudit corps flexible (21) adaptée pour couvrir ladite portion en forme de disque (20), ledit corps flexible ayant une forme de cavité et comprenant un bord libre (26) ajusté sur un bord périphérique (27) de la chambre d'aspiration (14).

5. Tire-lait selon les revendications 1 et 4, **caractérisé en ce que** ladite zone de contact (44) est une zone de bord de la portion en forme de disque (20) du corps rigide (19) de l'organe formant diaphragme (18).

6. Tire-lait selon la revendication 4, **caractérisé en ce que** ledit arbre (22) comporte une tête d'extrémité (36) supportée par un col (37) ayant une section plus étroite que le reste de l'arbre qui s'étend vers le haut à partir de la portion en forme de disque.

7. Tire-lait selon la revendication 6, **caractérisé en ce que** le levier d'actionnement comprend une tête (34) comportant un trou (35) adapté pour recevoir le col (37) de l'arbre (22).

8. Tire-lait selon la revendication 7, **caractérisé en ce que** ledit trou (35) est un trou allongé ayant une portion plus étroite (40) adaptée pour recevoir le col de l'arbre (22) pour déplacer l'organe formant diaphragme (18) et une portion plus large (41) pour recevoir la tête d'extrémité (36) de cet arbre (22) pour l'accouplement avec celui-ci.

9. Tire-lait selon la revendication 7, **caractérisé en ce que** ledit trou (35) est ouvert sur un côté et délimité par une portion de fourche (81) de la tête (34) du levier d'actionnement (33).

10. Tire-lait selon la revendication 1, **caractérisé en ce que** la chambre d'aspiration (14) est connectée au conduit (5) connecté à la portion en forme d'entonnoir (2) par l'intermédiaire d'un canal d'aspiration (13) qui est conçu pour transférer la variation de pression générée dans ladite chambre par le mouvement de l'organe formant diaphragme (18) audit conduit pour permettre au lait d'être exprimé à partir du sein ou acheminé vers le récipient.

11. Tire-lait selon la revendication 10, **caractérisé en ce que** ledit canal d'aspiration (13) s'ouvre dans la paroi de fond (48) de la chambre d'aspiration.

12. Tire-lait selon la revendication 10, **caractérisé en ce que** ledit conduit (5) et ledit canal d'aspiration (13) s'ouvrent dans une cavité (10A) d'une portion (10) du corps (1) à laquelle un clapet à lèvre est associé et à partir de laquelle le lait exprimé s'écoule dans le récipient (6) couplé au tire-lait.
